(19) Europäisches Patentamt
European Patent Office
Office européen des brevets

(11) Veröffentlichungsnummer: **0 218 248**
**A2**

(12) **EUROPÄISCHE PATENTANMELDUNG**

(21) Anmeldenummer: **86113966.5**

(51) Int. Cl.⁴: **C08F 8/00**

(22) Anmeldetag: **08.10.86**

(30) Priorität: **09.10.85 DE 3536077**

(43) Veröffentlichungstag der Anmeldung:
**15.04.87 Patentblatt 87/16**

(84) Benannte Vertragsstaaten:
**CH DE FR GB IT LI NL SE**

(71) Anmelder: **Ernst Mühlbauer KG**
**Fangdieckstrasse 61**
**D-2000 Hamburg 53(DE)**

(72) Erfinder: **Engelbrecht, Jürgen, Dr. Dipl.-Chem.**
**Petkumstrasse 8**
**D-2000 Hamburg 76(DE)**
Erfinder: **von Wallis, Helmut, Dr. Dipl.-Chem.**
**Hirschbergstrasse 2**
**D-2359 Henstedt-Ulzburg 2(DE)**
Erfinder: **Günther, Michael, Dr. Dipl.-Chem.**
**Eduard-Much-Strasse 39**
**D-2000 Hamburg 74(DE)**

(74) Vertreter: **Wolff, Hans Joachim, Dr.jur. Dipl.-Chem. et al**
**Beil, Wolff & Beil Rechtsanwälte Postfach 80 01 40 Adelonstrasse 58**
**D-6230 Frankfurt am Main 80(DE)**

(54) **Polymerisierbare Säure und Säurederivate enthaltende Verbindungen, dieselben enthaltende Mischungen und ihre Verwendung.**

(57) Die Erfindung betrifft oligomere oder prepolymere organische Verbindungen, die sowohl polymerisierbare ungesättigte Gruppen als auch Säurereste, deren Salze oder deren reaktive Derivatreste enthalten,

die ausgezeichnete Hafteigenschaften, insbesondere auf biologischen Substraten aufweisen und sich besonders im dentalen und medizinischen Bereich als polymerisierbare Haftvermittler, Haftfüllstoffe oder Haftzemente eignen.

EP 0 218 248 A2

# Polymerisierbare Säure und Säurederivate enthaltende Verbindungen, dieselben enthaltende Mischungen und ihre Verwendung

Die Erfindung betrifft polymerisierbare Säuregruppen oder Säuregruppenderivate enthaltende Verbindungen, dieselben enthaltende Mischungen und ihre Verwendung.

Derartige Verbindungen haften auf verschiedenen, besonders biologischen Substraten. Verbindungen dieser Art können für allgemeine Zwecke, aber besonders im dentalen und medizinischen Bereich als Komponenten für polymerisierbare Haftvermittler, Haftfüllungsmaterialien, Haftzemente und für ähnliche Mischungen eingesetzt oder zugesetzt werden.

Polymerisierbare Mischungen auf der Basis von monomeren, mit einer oder mehreren ungesättigten Gruppen versehenen Verbindungen bilden die Grundlage für eine Vielzahl von Kunststoffen, wobei im dentalen und medizinischen Bereich hauptsächlich Verbindungen mit Methacrylatgruppen wichtig sind. Mischungen dieser Art sind z.B. die Grundlage für Kunststoffüllungs-und Versiegelungsmaterialien.

Diese polymerisierbaren Mischungen können jedoch im allgemeinen keine chemische Verbindung mit anderen Materialien, besonders biologischen Substraten, ausbilden, es sei denn, diese Substrate enthalten noch selbst ausreichend unpolymerisierte polymerisierbare Gruppen.

Eine festere Verbindung kann daher nur über retentionsreiche Flächen, d.h. über einen Verbund rein mechanischer Art, z.B. durch Anätzen der Oberfläche von biologischen oder anorganischen Materialien erreicht werden. Durch Anwendung von Haftvermittlern, d.h. von Substraten, die mit biologischem oder anorganischem Material chemisch reagieren können als auch andererseits eine polymerisierbare Gruppe tragen, kann dieser Mangel jedoch behoben werden.

Es ist eine Reihe von solchen Haftvermittlern bekannt, z.B. die Vinyl-oder Methacrylgruppen tragenden Organosilane. Sie sind jedoch in ihrer Haftwirkung auf Siliciumdioxid, Siliciumdioxid enthaltende Gläser und Keramiken und Metalloxide, bzw. diese bildende Unedelmetalle beschränkt. Auf biologischen Substraten und besonders auf Zahn-oder Knochensubstrat zeigen sie keine Haftung, sondern wirken eher trennend.

Für Substrate solcher Art sind eine Reihe von polymerisierbaren Haftvermittlern mit anderen Haftgruppen gefunden worden. Da sind solche, die zum einen mit dem Collagen oder collagenähnlichen Anteilen dieser Substrate reagieren, wie z.B.

2-N-Allylamino-4,6-dichlor-1,3,5-triazin (US-PS 4 203 220), Kombinationen von Hydroxy-Methacrylester mit Dialdehyden (EP-0141324) oder Epoxymethacrylate.

Ferner gibt es eine Zahl von polymerisierbaren Verbindungen, die mit den Apatit-Verbindungen der Zahn-oder Knochensubstanz reagieren. Diese haftvermittelnden Verbindungen tragen Säuregruppen oder reaktive Säuregruppenderivate. Beispiele solcher polymerisierbarer Verbindungen sind;

ungesättigte organische Ester von Phosphor-oder Phosphonsäuren

(DE-AS 27 11 234, DE-OS 31 50 285);

ungesättigte organische Ester der Monofluorphosphorsäure (US-PS 3 997 504);

ungesättigte organische Ester von Säuren des Phosphors, die Chlor oder Brom direkt am Phosphor gebunden enthalten (EP 0 058 483);

ungesättigte organische Ester der Phosphorsäure, die als cyclische Pyrophosphate (Anhydride) vorliegen (DE-OS 30 48 410).

Es sind auch polymerisierbare Carbonsäuren und reaktive Carbonsäurederivate bekannt, die eine gute Haftung zur Zahnsubstanz zeigen, wie

4-Methacryloyloxyethyltrimellitsäure und deren Anhydrid (Takeyama, M. et al., I.Jap.Soc.f.Dent.App.a.Mat. 19, 179 (1978)) oder

Pyromellitsäure-bis-2-methacryloylethylester.

Ihre Anwendung ist jedoch sehr beschränkt. Es werden acetonische oder Methylmethacrylat-Lösungen verwandt.

In Dimethacrylatsystemen, wie z.B. in Mischungen mit Bis-GMA, treten Separation und Schwierigkeiten wie spontane Polymerisation oder keine Härtung auf, und die Beimischmengen müssen unter 5% bleiben (V.P. Thompson, IADR Symposium 1985, Publ. 1103).

Durch die aufgeführten polymerisierbaren Verbindungen ist in vielen Fällen eine mehr oder weniger starke Haftung von polymerisierbaren Füllungs- und Versiegelungsmaterialien an Zahn-und Knochensubstanz zu erreichen. Der Erfolg der Anwendung hängt auch weitgehend davon ab, in welcher Schichtdicke die haftvermittelnde Substanz aufgetragen wird, wie viele Haftgruppen mit dem biologischen Substrat und wie viele polymerisierbare Gruppen mit dem zu copolymerisierenden Material zur Reaktion kommen.

Der Erfindung liegt die Aufgabe zugrunde, Verbindungen zu finden, die an chemisch recht stabilen, molekularen Grundgerüsten sowohl mehrere polymerisierbare Gruppen als auch mehrere Haftgruppen gebunden enthalten.

Diese Aufgabe wird erfindungsgemäß gelöst durch oligomere oder prepolymere organische Verbindungen, die sowohl mehrere polymerisierbare ungesättigte Gruppen als auch mehrere Säurereste, deren Salze oder deren reaktive Derivatreste enthalten.

Es wurde gefunden, daß solche Verbindungen zu erhalten sind, wenn man Homo-oder Co-oligomerisate bzw. Homo-oder Co-polymerisate als oligomere oder polymere Grundgerüste herstellt, die die gewünschten Säuregruppen oder Säurederivatgruppen und bzw. oder funktionelle Gruppen enthalten, an die die gewünschten Säuregruppen oder Säurederivatgruppen und bzw. oder polymerisierbaren Gruppen aufgepfropft werden können.

Vorteilhafterweise enthalten die erfindungsgemäßen Verbindungen drei oder mehr polymerisierbare ungesättigte Gruppen und drei oder mehr Säurereste, deren Salze oder deren reaktive Derivate, wobei je nach Substrat, auf dem die Verbindungen haften sollen, und je nach polymerisierbarem System, in dem sie enthalten sind oder mit dem sie zusammen polymerisieren sollen, gewählt werden kann, ob mehr Säure-oder mehr polymerisierbare Gruppen oder beide Gruppen in gleicher Zahl vorhanden sein sollen.

Als polymerisierbare ungesättigte Gruppen eignen sich alle Alkenyl-, Alkenoxy-, Cycloalkenyl-, Aralkenyl-oder Alkenarylreste, vorteilhaft jedoch Acryl-, Methacryl-, Vinyl-oder Styryl-, und davon besonders vorteilhaft die Acryl-und Methacrylreste, die die polymerisierbaren Gruppen vieler Monomerer in Dentalmaterialien sind.

Als Säuregruppen der erfindungsgemäßen Verbindungen eignen sich prinzipiell all jene, die eine Haftwirkung auf oxidischen, mineralischen, keramischen, glasartigen, metallischen und biologischen Substraten zeigen können. Vorteilhafterweise werden jedoch Carbonsäurereste, folgende Reste von Säuren des Phosphors

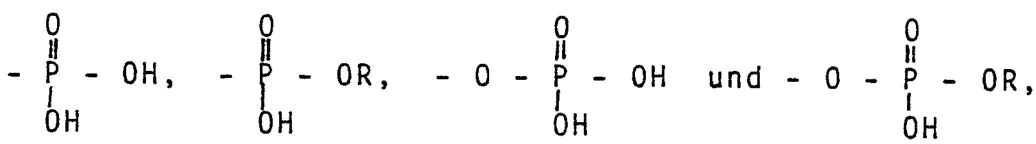

wobei R z.B. Alkyl , Aryl oder Vinyl bedeutet, folgende Reste von Säuren des Schwefels

$-SO_2H$, $-SO_3H$ oder $-O-SO_3H$,

oder folgende Reste von Säuren des Bors

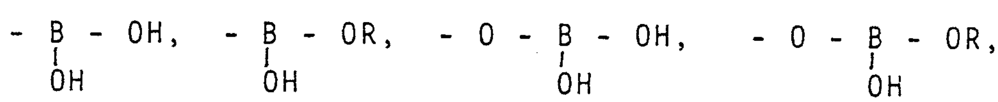

wobei R Alkyl , Aryl oder Vinyl bedeutet, eingesetzt.

Kationensäurereste wie $-NR_2H^+$ oder $-PR_2H^+$ - (R = H, Alkyl) sind ebenfalls geeignet.

Besonders vorteilhaft wird man jedoch die Carboxylat-, Phosphat-, Phosphonat-, Sulfonat-und Boratreste bzw. deren reaktive Derivate an das Oligomer-oder Polymergerüst knüpfen. Bei Derivaten mehrbasischer Säuren kann das Vorhandensein einer einzigen leicht zu hydrolysierenden Gruppe reichen, die anderen können stabil substituiert sein.

Die reaktiven Säurederivate der erfindungsgemäßen Verbindungen können Säurehalogenide, -anhydride, leicht zur Säure hydrolysierende Säureamide, -nitrile und -ester wie z.B. silyl-oder tert.-butylsubstituierte Verbindungen sein.

Die oligomeren oder polymeren Grundgerüste können linearer, verzweigter oder zyklischer Natur sein.

Sie können Polymerisate von ethylenisch ungesättigten Monomeren, aber auch oligomere oder polymere Verbindungen wie z.B. Polyester, Polyamide, Polyether, Polyphosphazene, Polysaccharide usw. sein, soweit ihr Gerüst ausreichend hydrolysestabil ist, sie andererseits mit den gewünschten polymerisierbaren Gruppen versehen werden können, sie die gewünschten Säuregruppen tragen oder sie mit diesen versehen werden können.

Die gewünschten Gruppen können aufgepfropft werden, wenn das Grundgerüst eine Vielzahl von funktionellen Gruppen gebunden enthält, die eine solche Pfropfreaktion erlauben, wie z.B. Alkohol-, Halogen-, Säurehalogenid-, Amino-, Epoxid-oder Isocyanatgruppen.

Das bedeutet, daß obengenannte Grundgerüste ungeachtet dessen, aus welchen Bausteinen sie aufgebaut sind, z.B. als Polyole, Polyhalogenide, Polysäurehalogenide, Polyamine, Polyepoxide oder Polyisocyanate oder als Mischung dieser vorliegen.

Bevorzugte Grundgerüste sind die Polymerisate aus ethylenisch ungesättigten Monomeren.

Dabei ist einerseits eine Gruppe von Monomeren geeignet, die zu Homo-oligomerisaten oder Homo-polymerisaten führt, andererseits eine Gruppe, die durch Kombination verschiedener Monomerer zu Co-oligomerisaten oder Co-polymerisaten führt. Aus der Gruppe der Homopolymerisate sind z.B. Oligo-oder Polymerisate von ungesättigten Säuren, die als Säurechlorid eingesetzt werden, geeignet (A):

(A)

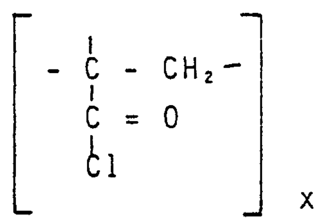

Sie können dann im ersten Schritt zu einem gewünschten Anteil mit z.B. Hydroxyethylmethacrylat umgesetzt und, falls die freie Säuregruppe gewünscht wird, darauf in einem zweiten Schritt der Rest der Säurechloride hydrolysiert werden. Die Verteilung der Gruppen erfolgt statistisch, z.B. -(B):

(B)

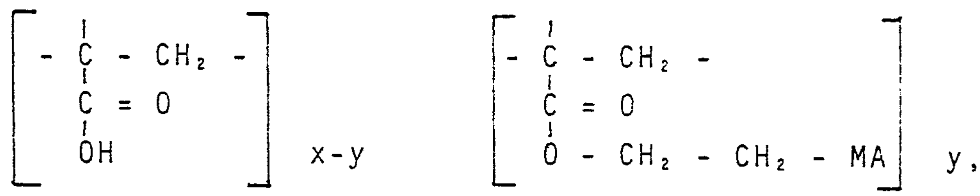

wobei MA einen Methacrylrest

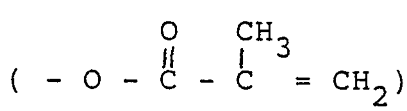

bedeuten soll.

Der zweite Schritt (Hydrolyse) kann jedoch auch durch einen Alkoholyseschritt mit säuregruppenhaltigen Alkoholen wie 1-Hydroxyethan-1,1-diphosphonsäure ersetzt werden, so daß Produkte wie (C)

(C) 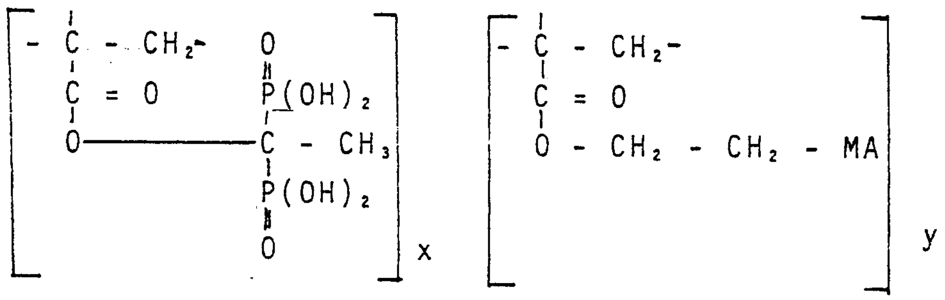

erhalten werden.

Ein weiteres gutes Grundgerüst für erfindungsgemäße Verbindungen sind Homopolymerisate aus ungesättigten Alkoholen (D). Ein Teil der Hydroxygruppen kann z.B. durch Veresterung mit einer ungesättigten Säure bzw. einem ungesättigten Säurechlorid mit polymerisierbaren Gruppen versehen werden; ein anderer Teil kann mit Säuren oder Säurechloriden wie z.B. Borsäure oder Phosphorylchlorid zu entsprechenden, erfindungsgemäßen Verbindungen (E, F) umgesetzt werden.

(D) 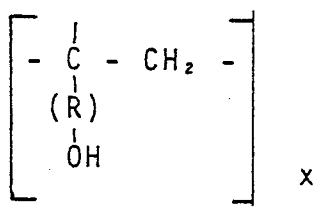

R, R' = kein oder inerter Rest

(E) 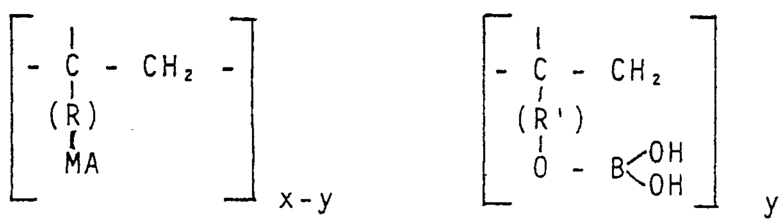

(F) 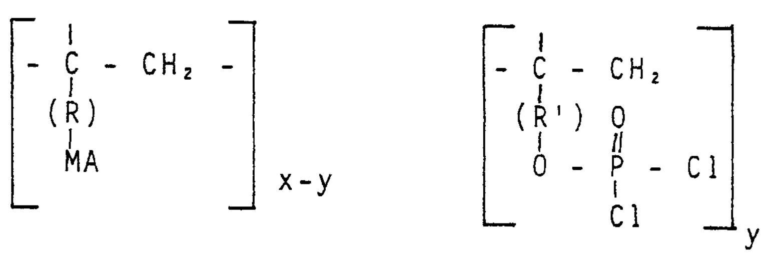

Liegt ein oligomeres oder polymeres Grundgerüst mit intramolekularen Anhydrid-oder intramolekularen Imidgruppen vor, können polymerisierbare Gruppen und Säuregruppen in einem Schritt angeknüpft werden.

Besonders bevorzugt sind Oligo-oder Polymerisate von Maleinsäureanhydrid:

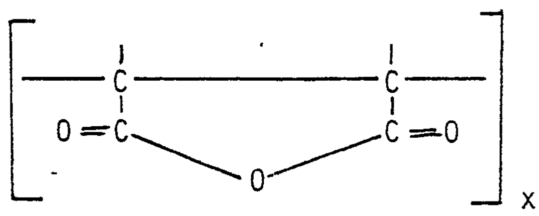

Dieses setzt sich z.B. mit einem Hydroxyalkylmethacrylat in Verhältnissen von 1:1 zu Produkten wie (G) um. Bei Zugabe von weniger Hydroxymethacrylat und dafür zusätzlichem Hydroxysäurederivat kann leicht ein erfindungsgemäßes Produkt mit 2 verschiedenen Haftgruppen erhalten werden (H),

(G) 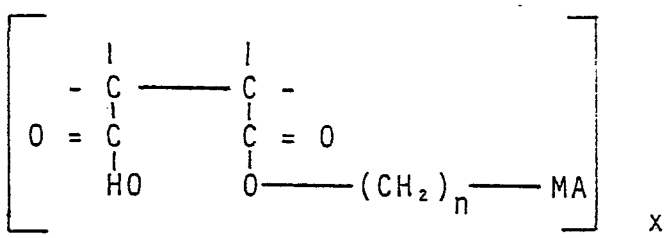 bzw.

(H) 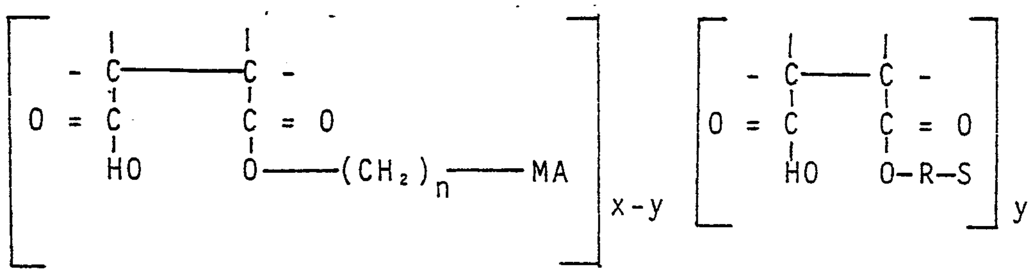

wobei S ganz allgemein der Säure-oder Säurederivatrest sein soll und R ein beliebiger Rest ist.

Bei einer weiteren Gruppe von bevorzugten Grundverbindungen, nämlich Co-oligomerisate oder Co-polymerisate, werden säure-oder säurederivathaltige Vinyl-, Styrol-oder (Meth)-acrylmonomere wie Vinylphosphat, Vinylphosphonat, Methacrylsäureester von Phosphorsäuren oder Phosphonsäuren, Styrylverbindungen mit Säuregruppen des Phosphors, Bors und Schwefel, wie z.B. sulfoniertes Styrol mit ungesättigten Verbindungen wie Vinylchloracetat oder chlormethyliertem Styrol copolymerisiert, so daß Verbindungen wie z.B. (I)

(I) 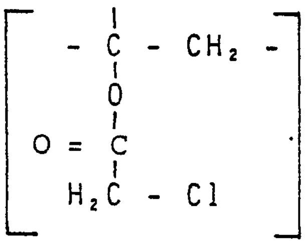 x 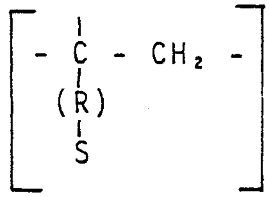 y

oder z.B. (K)

(K) 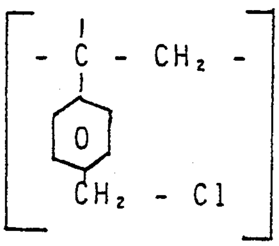 x 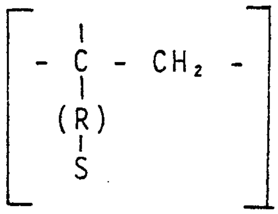 y

entstehen.

Verbindungen dieser Art können dann z.B. mit Natrium methacrylat in eine polymerisierbare erfindungsgemäße Verbindung (L) überführt werden:

(L) 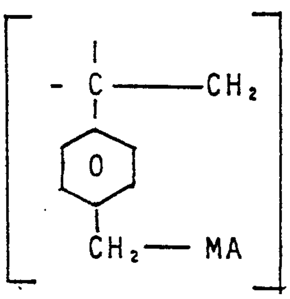 x 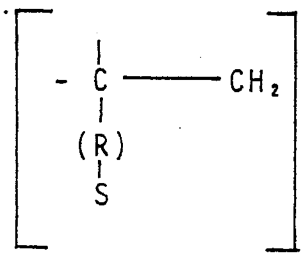 y

Auch Copolymerisate (M) von ungesättigten Alkoholen mit ungesättigten Säuren führen nach Reaktion mit Verbindungen wie z.B. Methacrylsäurechlorid zu erfindungsgemäßen Produkten - (N):

(M) 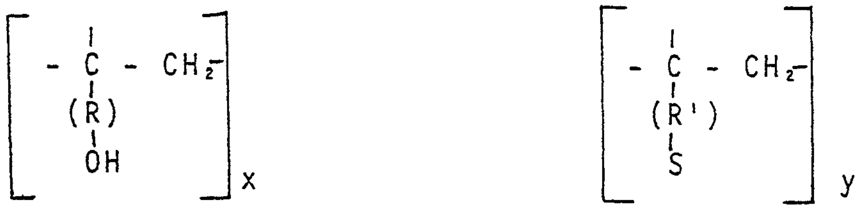

(N) 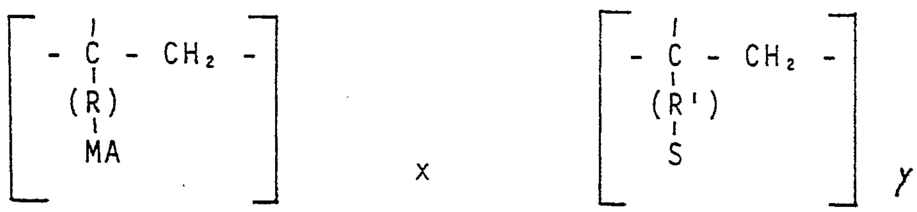

Beim Aufbau der Grundgerüste können auch Einheiten mit einpolymerisiert werden, die weder Säuregruppen tragen noch mit einer polymerisierbaren Gruppe versehen werden. Zum einen kann es nützlich sein, auf diese Weise die Löslichkeit zu verändern, wie z.B. beim Einbau von inerten Methylmethacrylat-Einheiten (O):

(O) 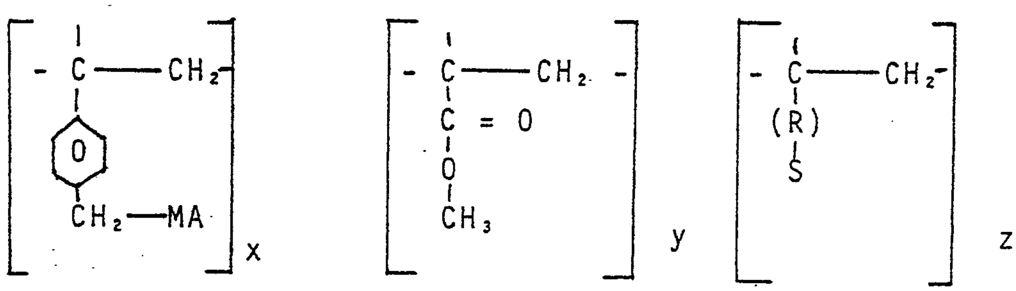

Zum anderen kann der Einbau von zusätzlichen Einheiten mit Halotriazin-, Epoxid-, Isocyanat-oder Aldehydgruppen (P) zum Zwecke einer zusätzlichen Reaktion mit dem Collagenanteil von Zahn-oder Knochensubstrat von Nutzen sein.

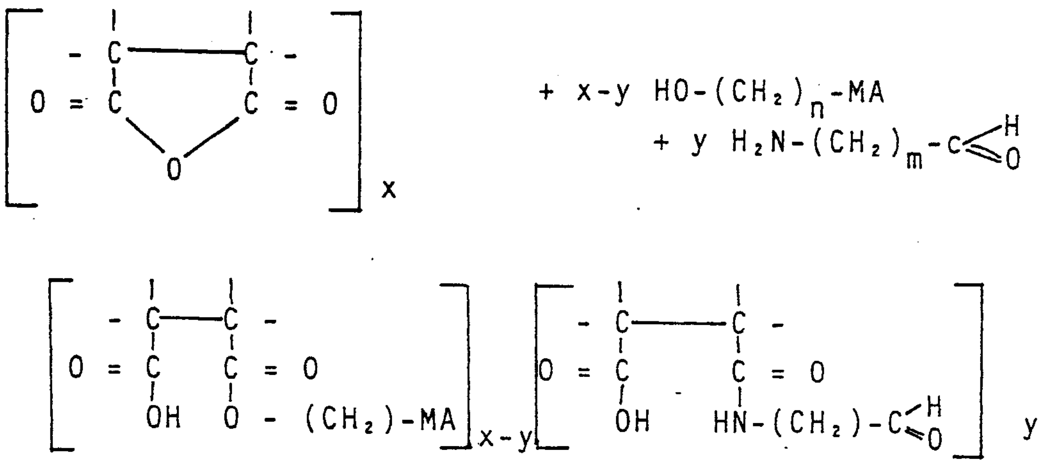

(P)

Auch der Einbau von Einheiten mit Gruppen, die Teile eines Polymerisations-Katalysatorsystems sein können, kann von Vorteil sein, wobei diese Gruppen nicht unbedingt bei der Homo-oder Co-Polymerisation des Grundgerüstes als solche Gruppen vorhanden sein müssen, sondern auch nachträglich z.B. über eine Halogen-, Alkohol-, Anhydrid-oder Amin-funktionelle Gruppe angeknüpft werden können.

Die Art der Reaktionsführungen, die zu den oligomeren oder prepolymeren Grundverbindungen führen, kann durch die Wahl des Lösungsmittels, der Löslichkeit, der Konzentration, der Temperaturen und die Wahl der Polymerisationskatalysatoren bestimmt werden und sind dem Fachmann bekannt.

Wichtig kann sein, daß die benutzten Polymerisationskatalysatoren durch die Reaktion selbst zerstört werden oder eventuelle Reste von diesen vor dem Einbringen der polymerisierbaren Gruppen der erfindungsgemäßen Verbindungen entfernt werden.

Die erfindungsgemäßen oligomeren Verbindungen haben vorteilhafterweise ein Molekulargewicht von größer als 500, die prepolymeren Verbindungen haben vorteilhafterweise ein Molekulargewicht von größer als 1.500, bevorzugt nicht größer als 100.000, besonders bevorzugt nicht größer als 20.000.

Die erfindungsgemäßen Verbindungen können für sich allein, bevorzugt jedoch als Mischung mit anderen poly merisierbaren Verbindungen verwendet werden.

So können die Mischungen noch polymerisierbare ungesättigte Monomere, Oligomere oder Polymere enthalten, die keine Säuregruppen, deren Salze oder deren reaktive Derivatgruppen aufweisen.

Besonders eignen sich jene Monomere, die Bestandteile üblicher polymerisierbarer Composites und polymerisierbarer Dentalharzmischungen sind, wie z.B. Bis-GMA oder Triethylenglykoldimethacrylat.

Ebenso können die Mischungen Beimengungen von bekannten monomeren polymerisierbaren ungesättigten Verbindungen enthalten, die Säuregruppen, deren Salze oder deren reaktive Säurederivate aufzeigen. Auch solche polymerisierbaren Verbindungen, die Gruppen tragen, welche eine besondere Haftung am Collagen des Dentins zeigen, und wie sie in den vorstehend genannten Druckschriften beschrieben sind, sind ein bevorzugter Zusatz.

Ebenso können die Mischungen gegebenenfalls noch andere Verbindungen enthalten, die zwar Säuregruppen, deren Salze oder deren reaktive Säurederivatgruppen aufweisen, aber keine Gruppen enthalten, die polymerisierbar sind. In diesem Falle werden mehrbasische Säuren wie Weinsäure, Citronensäure, Mellitsäure, Polycarbon-, Polyphosphor-, Polyphosphon-oder Polysulfonsäuren, aber auch Chelatbildner, wie z.B. Ethylendiamintetraessigsäure, bevorzugt sowie auch deren Salze.

Auch Zusätze von Anhydriden mehrbasischer Säuren, wie z.B. Pyromellitsäuredianhydrid oder 1,2,3,4-Cyclopentantetracarbonsäure können von Vorteil sein, besonders, wenn bei Zweikomponentenmischungen eine Alkoholverbindung zugesetzt ist. Hilfsweise kann auch ein leichtflüchtiges Lösungsmittel zugesetzt sein, um einen besonders dünnen Auftrag der Mischung auf dem Gegenstand, auf dem sie haften soll, zu erreichen. Zudem kann dieser Zusatz sinnvoll sein, wenn eine oder mehrere Komponenten zum Zwecke der Lagerung getrennt gehalten werden müssen oder in den erfindungsgemäßen Verbindungen oder sie enthaltenden Mischungen nicht ausreichend löslich sind.

Als Polymerisationskatalysatoren eignen sich prinzipiell alle diejenigen Systeme, die eine radikalische Polymerisation von olefinischen Verbindungen auslösen können. Dabei ist es nicht wesentlich, ob die Katalysatorreaktion durch Erhitzen, durch Hinzufügen eines den Katalysator zur Reaktion bringenden Aktivators oder durch Bestrahlen mit Licht initiiert wird. Vielmehr ist es wichtig, daß das Katalysatorsystem ausreichend löslich in der Mischung ist und nicht wesentlich durch Säure-oder Säurederivatgruppen enthaltenden polymerisierbare Verbindungen blockiert oder zum Zerfall gebracht wird.

Bevorzugt werden für lichthärtbare Mischungen Härtungssysteme aus alpha-Diketonen und tertiären Aminen, wie sie z.B. in der FR-PS 21 56 760 beschrieben sind, oder Kombinationen von Sulfinsäuresalzen und Xanthonen oder Thioxanthonen, wie sie z.B. in EP 0 132 318 beschrieben sind.

Im Falle von 2-Komponenten-Mischungen eignen sich besonders Kombinationen von einer Komponente, die organische Perodixe enthält und von einer anderen Komponente, die ein tertiäres Amin und eine Verbindung, die Schwefel in der Oxidationszahl +2 oder +4 aufzeigt, enthält.

Die Komponente, die nicht das Peroxid enthält, kann auch bevorzugt zweiwertige Metallionen enthalten, insbesondere des Calciums und besonders, wenn Tertiärbutylpermaleinat als Peroxid verwendet wird.

Besonders bevorzugt sind Benzoylperoxid als organisches Peroxid und Natriumbenzolsulfinat oder Natrium-paratoluol-sulfinat als Schwefelverbindungen.

Eine besonders vorteilhafte Mischung liegt vor, wenn der Komponente, die das Natriumsulfinat enthält, zum ausreichenden Lösen dieses Salzes Alkohole zugegeben werden, die eine oder mehrere polymerisierbare olefinische ungesättigte Gruppen enthalten.

Dafür eignen sich z.B. Hydroxyalkylmethacrylate wie Hydroxyethylmethacrylat oder Hydroxygruppen tragende Vinylverbindungen, wie Allylalkohol, und besonders hydroxylgruppenhaltige Dimethacrylatverbindungen, wie Bisphenol-A-glycidylmethacrylat oder Glycerindimethacrylat oder hydroxylgruppenhaltige Divinylverbindungen wie Glycerindiallylether.

In der Regel ist eine Zugabe von 10 bis 20% dieser hydroxylgruppenhaltigen polymerisierbaren Monomeren notwendig. Ebenso können geringe Mengen Schwermetallsalze wie z.B. von Fe, Cu, Mn, Co, Sn, Cr, Ni und Zn zugegeben sein, die sich günstig auf das Haftverhalten z.B. auf Zahnsubstanz auswirken.

Auch heilende Komponenten wie z.B. Cortison oder Cortikoide, Klauenöl oder andere können zugegeben werden, sind dann aber aus rein medizinischen Gründen indiziert. Aus ähnlichen Gründen können auch Fluorid spendende Verbindungen, wie z.B. Natriumfluorphosphat oder Aminfluoride, zugesetzt sein.

Die die erfindungsgemäßen Verbindungen enthaltenden Mischungen können auch mit gegebenenfalls oberflächenbehandelten anorganischen oder organischen Füllstoffen versetzt sein, wobei der anorganische Füllstoff aus der Gruppe der für Composites üblichen Mehle von Quarz, mikrofeiner Kieselsäure, Aluminiumoxid, Bariumgläsern und anderen mineralischen (inerten) Substanzen, möglichst in silanisierter Form, sein kann, aber auch aus der für die erfindungsgemäßen Verbindungen vorteilhaften Gruppe von feinteiligen Füllstoffen wie Pulver von Metalloxiden, Metallhydroxiden, reaktiven Gläsern und Keramiken, Zeolithen, Unedelmetallen, Apatith usw., die mit den Säuregruppen, Salzen oder Säurederivatgruppen der erfindungsgemäßen polymerisierbaren Verbindungen eine Bindung eingehen können.

Die erfindungsgemäßen Verbindungen sind neu und ihre Eigenschaften können leicht durch Variationen der Verhältnisse von polymerisierbaren Gruppen zu haftenden Säure-bzw. Säurederivatgruppen als auch durch die Wahl der Molekülgröße modifiziert werden.

Es können eine Vielzahl Verbindungen mit hervorragenden Hafteigenschaften hergestellt werden.

Für sich allein oder in Mischungen mit anderen polymerisierbaren Verbindungen können sie mit verschiedensten Füllstoffen gefüllte, nach der Aushärtung mechanisch sehr feste und auf verschiedensten Untergründen haftende Werkstoffe sein. Selbst bei Gehalten von 1 bis 10% an erfindungsgemäßen Verbindungen zeigen die polymerisierbaren Mischungen, gefüllt oder ungefüllt, nach der Polymerisation eine deutliche Haftwirkung oder Haftverbesserung auf verschiedenen oxidischen, mineralischen, keramischen, glasartigen; metallischen oder biologischen Substraten sowie einen sehr festen Verbund mit Composites, sofern diese kurz davor oder kurz danach aufpolymerisiert sind.

Besonders für die Haftung an Zahn-und Knochensubstanz ergeben sich bessere Möglichkeiten von sicherer anzuwendenden Haftmischungen und dauerhafteren festeren Verbindungen von Versiegelungs-und Füllungsmaterialien als Haftvermittler zu denselben und als Kleber zwischen diesen Substraten.

Auch die Einarbeitbarkeit von Füllstoffen in polymerisierbare Mischungen kann durch erfindungsgemäße Verbindungen verbessert werden und zu höherer Festigkeit führen.

Auch ausgehärtete Produkte können bevorzugt Anwendung finden. Sie zeigen weiterhin noch reaktionsfähige, zwar nicht mehr polymerisationsfähige, so doch chemisch kupplungsfähige Oberflächen.

Das gleiche trifft auch für ausgehärtete Filme auf Pigmenten oder Füllstoffen zu. Sie sind am und um den Füllstoff gebunden, machen den eigentlichen Füllstoff inert und können aber doch noch z.B. in Zementsystemen mit Hilfe der Säuregruppen mit oxidischen Komponenten abbinden.

Nachstehende Beispiele dienen der Erläuterung der Erfindung. Außer wenn etwas anderes angegeben ist, beziehen sich Teile und %-Angaben auf das Gewicht.

Beispiel 1

Herstellung einer prepolymeren polymethacrylierten Polyacrylsäure:

86 g wasserfreie Polyacrylsäure mit einem Molgewicht von ca. 5.000 wurden mit 130 g frischdestilliertem Thionylchlorid, 300 ml Tetrahydrofuran und 200 ml Dioxan versetzt und unter Rückfluß gekocht, bis die Gasentwicklung beendet war. Der Überschuß an Thionylchlorid wurde dann vollständig abdestilliert und nochmals mit 100 ml Dioxan versetzt. Darauf wurden 120 g Triethylamin zugegeben und mit 135 g Hydroxyethylmethacrylat unter Eiskühlung langsam versetzt und eine Woche stehengelassen. Triethylamin und Tetrahydrofuran wurden abdestilliert und die verbleibende Dioxanlösung durch Zugabe von 10 ml Wasser hydrolysiert. Danach wurden alle flüchtigen Anteile abgezogen, der zurückbleibende Rest mit Ethanol extrahiert und anschließend die ethanolische Lösung eingedampft und mit Hexan gewaschen. Man erhielt 42 g eines etwas viskosen rötlichen Öls.

IR-spektroskopisch wurden olefinische Doppelbindungen (1.640 $cm^{-1}$) und Carbonsäurereste festgestellt. Das Produkt reagiert deutlich sauer (pH 3,5) und vergelt leicht bei Zusatz von Benzoylperoxid/Aktivator.

Beispiel 2

Herstellung einer polymerisierbaren, haftvermittelnden Mischung:

Es wurde ein Harz hergestellt aus:

50 Teilen Bisphenol-A-glycidyldimethacrylat (Bis-GMA)

50 Teilen Triethylenglykoldimethacrylat (TEDMA)

5 Teilen des Produktes von Beispiel 1

1 Teil Benzoylperoxid

sowie eine Lösung aus

96 Teilen Ethanol

3 Teilen Natriumbenzolsulfinat

1 Teil N,N-Bishydroxyethyl-p-toluidine

Es wurden Rinderzähne wie in EP 0 058 483 beschrieben präpariert, dann je 2 Tropfen des Harzes und der Lösung kräftig zusammengemischt (20 sec), auf eine ungeätzte Dentinfläche des Zahnes aufgepinselt, getrocknet und das handelsübliche Composite "Composite Merz" darauf in einer Form von 5 x 5 mm aushärten lassen.

Nach 24-stündiger Lagerung wurden die Füllungsmaterialien mit einer Zugprüfmaschine vom Zahn abgezogen und ein Durchschnittswert von 3,57 $N/mm^2$ gemessen, bei dem die Proben abrissen.

Beispiel 3

Als Vergleich gegenüber Beispiel 2 wurde ein Harz hergestellt aus:

50 Teilen Bis-GMA

50 Teilen TEDMA

sowie

1 Teil Benzoylperoxid

und nach Mischen mit der Lösung aus Beispiel 2 ebenfalls auf gleich präparierte Zähne aufgepinselt, getrocknet und mit "Composite Merz" zusammen aushärten gelassen.

Nach gleicher Lagerung wurde beim Abzugsversuch ein Wert von nur 0,80 $N/mm^2$ gemessen.

Beispiel 4

Herstellung einer polymethacrylierten Oligomaleinsäure:

260 g Maleinsäureanhydrid wurden mit 2.000 ml Toluol und 40 g Benzoylperoxid unter Rückfluß 6 Tage erhitzt. Es bildete sich ein bräunlich oranger Niederschlag. Nach Beendigung der Reaktion

wurde die überstehende Toluol-Lösung dekantiert, der Rückstand mit Hexan gewaschen. Ausbeute: 200 g. Diese 200 g wurden mit der gleichen Menge Tetrahydrofuran versetzt.

Eine Molekulargewichtsbestimmung ergab ein mittleres Molekulargewicht von 439, was in etwa einem Oligomerisationsgrad von 4 Maleinsäureanhydrid-Einheiten entspricht.

Das IR-Spektrum zeigte die C=O-Bande von Anhydrid-Gruppen (1.790 $cm^{-1}$), jedoch keine Säure-OH und keine Doppelbindungen. 100 g der Tetrahydrofuranlösung des Oligomaleinsäureanhydrids wurden mit 10 g Zinkstaub versetzt, gerührt und wieder abfiltriert, wodurch die Farbe der Lösung deutlich hell wurde.

Nach Zugabe von 60 g Hydroxyethylmethacrylat und katalytischen Mengen Orthophosphorsäure wurde der Ansatz für 2 Wochen stehengelassen. Die Mischung wurde deutlich viskoser. Nach Abziehen der flüchtigen Komponenten im Vakuum und Waschen in Hexan erhielt man ein viskoses Öl, das sehr gut in Aceton, aber auch in TEDMA und Bis-GMA löslich war.

Das IR-Spektrum zeigte, daß die Anhydrid-C=O-Bande fast verschwunden war, eine Säure-OH-Bande und auch eine Doppelbindungsbande deutlich erschienen war.

Beispiel 5

Eine haftende, lichthärtende Mischung - (Haftcomposite) wurde bereitet aus:

50 Teilen Bis-GMA

50 Teilen TEDMA

1 Teil Campherchinon

1 Teil Dimethylaminoethylmethacrylester

1 Teil Butyldimethylanilin

50 Teilen des Produktes von Beispiel 4 und

150 Teilen feinteiliges Siliciumdioxid, silanisiert.

Die Mischung war dünnfließend pastös und polymerisierte bei Bestrahlung mit Halogenlicht binnen 40 sec zu einem sehr harten und bruchfesten Material aus. Es zeigte ohne vorherige Anwendung von Haftvermittlern Haftwerte am Dentin im Durchschnitt von 2,5 $N/mm^2$. Übliche Composites wurden mit ca. 0,2-0,6 $N/mm^2$ gemessen.

Beispiel 6

Herstellung einer polymethacrylierten Polycarbonpoly phosphonsäure:

100 g der Tetrahydrofuranlösung des Oligomaleinsäureanhydrids von Beispiel 4 wurden mit Zinkstaub gerührt und mit 30 g Hydroxyethylmethacrylat versetzt.

Nachdem die Mischung zwei Wochen bei Raumtemperatur reagiert hatte, wurden 40 g Hydroxy-ethan-1,1-disphosphonsäure hinzugelöst und weitere zwei Wochen stehengelassen.

Nach Abziehen des Tetrahydrofurans erhielt man eine recht viskose Flüssigkeit, die mit Hexan gewaschen wurde. Das IR-Spektrum zeigte C=C-Banden bei 1.640 $cm^{-1}$, P(O)OH-Banden bei 1.200 $cm^{-1}$. Die Substanz reagiert sauer und vergelt bei Zugabe von Aktivator/Peroxid.

Beispiel 7

Ein härtbares, haftvermittelndes Harz aus:

50 Teilen Bis-GMA

50 Teilen TEDMA

10 Teilen des Produktes von Beispiel 6 und

1 Teil Benzolyperoxid

wurde mit der Lösung aus Beispiel 1 gemischt und auf Dentin aufgetragen, wie in Beispiel 1.

Analog wurde mit dem Dentinhaftmittel "Scotch Bond" (3M) verfahren und beide mit dem "Composite Merz" zusammen ausgehärtet und nach 24 h die Zugfestigkeit gemessen.

Während bei "Scotch Bond" 2,7 $N/mm^2$ gemessen wurde, erhielt man bei der Mischung mit der erfindungsgemäßen Komponente einen Wert von 3,8 $N/mm^2$.

Beispiel 8

Herstellung eines prepolymeren polymethacrylierten Polychlorophosphates:

42 g Hydroxyethylmethacrylat und 8 g Lauroylperoxid wurden in 400 ml Toluol gelöst, 1 h bei 65°C gehalten. Das entstandene Pulver wurde abfiltriert, mit Hexan gewaschen und getrocknet.

Ausbeute: 40g Polyhydroxyethylmethacrylat - (Poly-HEMA) Molgewichtsbestimmung: 5.700 ≙ ca. 44 Monomereinheiten

IR-spektroskopisch identisch mit höhermolekularem Poly-HEMA-Produkt der Firma Aldrich

13 g des selbst hergestellten Poly-HEMA wurden mit 8 g Methacrylsäurechlorid und 8 g Triethylamin 3 Tage gerührt, der Niederschlag mit Wasser gewaschen und getrocknet.

Ausbeute: 15 g teilmethacryliertes Poly-HEMA

3,3 g dieses Pulvers werden in 50 ml Tetrahydrofuran mit 1,5 g Phosphorylchlorid versetzt und 4 Tage bei Raumtemperatur gerührt. Nach Filtration des Niederschlages und Waschen mit Hexan werden 3,8 g eines weißen Pulvers erhalten, welches IR-spektroskopisch gut mit einem polymethacrylierten Produkt mit $-O-P(O)Cl_2$-Gruppen in Einklang gebracht werden kann. Das Produkt zeigt kaum noch C-OH-Banden, jedoch weiterhin die C=O (1 730 $cm^{-1}$) und C=C-Banden (1 640 $cm^{-1}$) der Methacrylgruppe sowie neu aufgetretene Banden im Bereich P-O-Alkyl (1 030 $cm^{-1}$).

Beispiel 9

Herstellung einer halogenlichthärtenden Dentinhaftmischung:

55 Teile Bis-GMA

45 Teile TEDMA

5 Teile polymethacryliertes Polychlorophosphat aus Beispiel 8

1 Teil Butyldimethylanilin

1 Teil Campherchinon

wurden gemischt, sehr dünn auf Rinderdentin - (behandelt wie in Beispiel 2) aufgetragen und zusammen mit zylindrischen Proben von "Composite Merz lichthärtbar" (Durchmesser 4 mm, Höhe 6 mm) während 2 min bei Halogenlicht ausgehärtet.

Nach 24-stündiger Lagerung in Wasser zeigten die Proben im Zugversuch eine mittlere Haftfestigkeit am Dentin von 4,5 $N/mm^2$.

Ein Vergleichsversuch mit einer Dentinhaftmischung ohne Zusatz des polymethacrylierten Polychlorophosphats, nämlich

55 Teile Bis-GMA

45 Teile TEDMA

1 Teil Butyldimethylanilin

1 Teil Campherchinon

wurde unter gleichen obigen Bedingungen angewandt, zeigte jedoch nur eine mittlere Haftfestigkeit von 0.48 $N/mm^2$.

Beispiel 10

Herstellung von polymethacrylierter Polyborsäure:

3, 3 g teilmethacryliertes Poly-HEMA aus Beispiel 8 wurden mit 3, 1 g Borsäure und 4, 1 g Phosphorsäure so lange auf 80°C in Dioxan erhitzt, bis die Niederschlagsbildung abgeschlossen war.

Nach Filtration wurde mit Wasser phosphat-und borsäurefrei gewaschen, getrocknet und 3,45 g einer leicht bräunlichen polymethacrylierten Polyborsäure erhalten.

IR-spektroskopisch sind die C=C-Banden und C=O-Banden unverändert, es treten neue Banden (B-OH) bei 3.220 $cm^{-1}$ im Spektrum auf. Der Borgehalt ergibt sich zu 2,4 Gew. -%.

Beispiel 11

Bereitung einer dentinhaftenden 2-Komponenten-Mischung:

Es wird ein Aktivatorharz bestehend aus

55 Teilen Bis-GMA

45 Teilen TEDMA

3 Teilen N,N-Bishydroxyethyl-p-toluidin

und ein Katalysatorharz bestehend aus

55 Teilen Bis-GMA

45 Teilen TEDMA

3 Teilen Benzoylperoxid

5 Teilen polymethacrylierte Polyborsäure Beispiel 10 zu gleichen Teilen gemischt, dünn auf Dentin aufgetragen und zusammen mit einer zylindrischen Probe "Composite Merz" ausgehärtet (I).

Ein Vergleichsversuch wurde mit einer Mischung aus Aktivator-und Katalysatorharz durchgeführt, wobei die Polymethacrylpolyborsäure-Komponente weggelassen wurde (II). Nach 24-stündiger Lagerung in Wasser zeigen sich große

Unterschiede in der Haftung an Dentin:

mittlere Zugfestigkeit der Proben I: 7,82N/mm²

mittlere Zugfestigkeit der Proben II: 0,54N/mm²

Beispiel 12

Herstellung eines polymethacrylierten Polysulfonats:

5,4 g Hydroxyethylmethacrylat, 10,1 g Kaliummethacryloylpropylsulfonat und 1,6 g Lauroylperoxid wurden in 80 ml Methanol und 20 ml Toluol bei 65°C erhitzt, bis die Niederschlagsbildung beendet war. Es wurde filtriert, mit Hexan gewaschen und getrocknet.

Ausbeute: 6,4 g wasserlösliches Copolymerisat, Molgewicht 7.490, entsprechend ca. 20 Einheiten von jedem der beiden eingesetzten Monomeren.

1,88 g des Copolymerisates wurden mit 0,54 g Methacrylsäurechlorid und 0,50 g Triethylamin in 50 ml Tetrahydrofuran vier Tage bei Raumtemperatur gerührt. Der entstandene Niederschlag wurde mit Hexan gewaschen und getrocknet.

Ausbeute: 1,92 g weißes, wasserlösliches, pulveriges Polymethacrylpolykaliumsulfonat, welches im IR deutlich wieder C=C bei 1.640 $cm^{-1}$ zeigt.

Gehalt an Kalium: 7,9%

Gehalt an Schwefel: 6,2%

Beispiel 13

Herstellung einer lichthärtenden Haftmischung:

Eine Mischung von

55 Teilen Bis-GMA

45 Teilen TEDMA

1 Teil Butyldimethylanilin

1 Teil Campherchinon

5 Teilen Polymethacrylpolykaliumsulfonat aus Beispiel 12

wird wie in Beispiel 9 auf Rinderdentin getestet. Es ergibt sich eine mittlere Haftfestigkeit von 5,8 N/mm².

Beispiel 14

Haftvermittler zwischen Composites auf Methacrylat-Basis und oxidischem, mineralischem, keramischem, glasartigem und metallischem Substrat:

Eine lichthärtbare Mischung aus

50 Teilen Bis-GMA

50 Teilen TEDMA

10 Teilen polymethacrylierter Oligomaleinsäure aus Beispiel 4

1 Teil Butyldimethylanilin

1 Teil Campherchinon

wurde dünn auf eine fein geschliffene Fläche der verschiedenen unten angeführten Substrate aufgetragen und jeweils mit Zylindern (4 mm Durchmesser, 6 mm Höhe) von "Composite Merz lichthärtbar" zusammen 2 min mit Halogenlicht - (450-500 nm) bestrahlt.

Nach 24 h Wasserlagerung bei 37°C wurden folgende Haftfestigkeiten im Zugversuch ermittelt. - (In Klammern die Werte eines Vergleichsversuchs mit gleicher Haftmischung, jedoch ohne erfindungsgemäße Komponente)

| | N/mm² | |
|---|---|---|
| Ionomer Cement | 5,2 | (1,2) |
| Silicat Cement | 17,8 | (0,9) |
| Glas | 12,1 | (0,3) |
| Porzellan | 19,9 | (0,1) |
| Co-Cr-Legierung | 9,2 | (0,9) |
| Au-Pt-Legierung | 3,6 | (0,2) |

Beispiel 15

Herstellung einer prepolymeren polymethacrylierten Polymaleinsäure:

60 g Maleinsäureanhyerid und 9 g Lauroylperoxid wurden in 150 ml Tetrahydrofuran vier Tage unter Rückfluß gekocht, das Tetrahydrofuran wieder abgezogen und das viskose Öl mit Hexan gewaschen (Polymaleinsäureanhydrid, Molekulargewicht, 1.850, entsprechend ca. 17 Einheiten, IR-Spektrum identisch mit dem Oligomaleinsäureanhydrid aus Beispiel 4).

9,8 g des Öls wurden in 30 ml THF gelöst und mit 12 g Hydroxymethacrylat zwei Wochen gerührt. Nach Abziehen des THF erhielt man ein viskoses Öl von polymethacrylierter Polymaleinsäure, dessen IR-Spektrum identisch mit dem der polymethacrylierten Oligomaleinsäure aus Beispiel 4 war.

Beispiel 16

Haftvermittler:

Eine Mischung von

50 Teilen Bis-GMA

50 Teilen TEDMA

10 Teilen polymethacrylierter Polymaleinsäure aus Beispiel 15

1 Teil Butyldimethylanilin

1 Teil Campherchinon

wird analog Beispiel 9 an Rinderdentin getestet. Mittlere Haftfestigkeit 6,8 N/mm².

Beispiel 17

Herstellung einer prepolymeren Polymethacrylpolyaldehydopolymaleinsäure:

9, 8 Polymaleinsäureanhydrid werden in 30 ml THF gelöst, mit 6 g Hydroxyethylmethacrylat versetzt und zwei Wochen in Gegenwart von HCl-Gas gerührt, bis kein freies Hydroxyethylmethacrylat mehr festgestellt werden kann. Darauf werden 7,5 g Aminobutyraldehyddiethylacetal und etwas Triethylamin zugegeben und nochmals drei Tage gerührt.

Vom weißen Niederschlag wurde abfiltriert, das THF abgezogen und das zurückbleibende Öl mit Hexan, verdünnter HCl und Wasser gewaschen und getrocknet. Das Öl (Polymethacrylpolyaldehydopolymaleinsäure) reduziert ammoniakalische Silberlösung und sein IR-Spektrum zeigt C=C-Banden, COOH-Banden und keine Anhydridbanden mehr.

Beispiel 18

Haftvermittler:

Eine Mischung von

50 Teilen Bis-GMA

50 Teilen TEDMA

10 Teilen polymethacrylierter Polyaldehydopolymaleinsäure

1 Teil Butyldimethylanilin

1 Teil Campherchinon

wird analog Beispiel 9 am Rinderdentin getestet.

Mittlere Haftfestigkeit 9,5 N/mm².

Beispiel 19

Haftvermittler für Fingernägelmaterial:

Jeweils 5 Fingernägel werden zum einen mit einem dünnen Film des Haftvermittlers aus Beispiel 16 (Gruppe 1) und zum anderen mit einem dünnen Film einer entsprechen den Mischung (Gruppe 2), in der die Haftkomponente "polymethacrylierte Polymaleinsäure" weggelassen ist, versehen und darauf mit etwa 1 mm Schichtdicke des lichthärtenden, dünnfließenden Composite "Liquicoat" der Fa. Merz in einer Fläche von ca. 2 x 2 mm zusammen ausgehärtet.

Während die Composite-Schichten der Gruppe 2 schon nach 2 Tagen vollständig abgefallen sind, haften 4 Proben der Gruppe 1 noch nach 2 Wochen auf dem Fingernagel.

## Ansprüche

1. Oligomere oder prepolymere organische Verbindungen, die an ein oligomeres oder prepolymeres Grundgerüst mehrere polymerisierbare ungesättigte Gruppen und mehrere Säurereste, deren Salze oder deren reaktive Derivate gebunden enthalten.

2. Verbindungen nach Anspruch 1, dadurch gekennzeichnet, daß sie mindestens drei polymerisierbare ungesättigte Gruppen und mindestens drei Säurereste, deren Salze oder deren reaktive Derivatreste enthalten.

3. Verbindungen nach einem der Ansprüche 1 oder 2, dadurch gekennzeichnet, daß die polymerisierbaren ungesättigten Gruppen Acryl-, Methacryl-, Vinyl-, Styryl-oder eine Mischung derselben bedeuten.

4. Verbindungen nach einem der Ansprüche 1 oder 2, dadurch gekennzeichnet, daß die polymerisierbaren ungesättigten Gruppen Acryl-, Methacryl-oder eine Mischung derselben bedeuten.

5. Verbindungen nach Anspruch 1 bis 4, dadurch gekennzeichnet, daß die Säuregruppen Carbonsäurereste, folgende Reste von Säuren des Phosphors

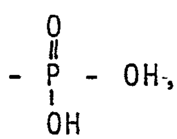 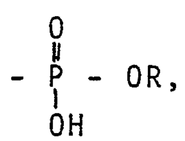 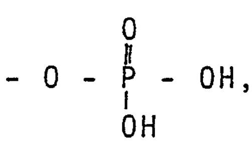 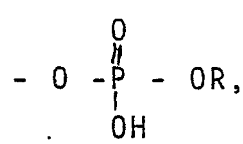

wobei R Alkyl, Aryl oder Vinyl bedeutet, folgende Reste von Säuren des Schwefels

$-SO_2H$, $-SO_3H$, $-O-SO_3H$,

oder folgende Reste von Säuren des Bors

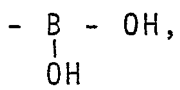 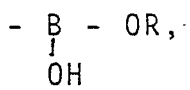 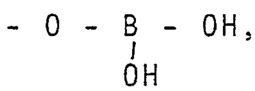 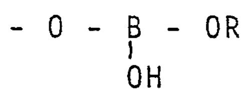

worin R Alkyl, Aryl oder Vinyl bedeutet, sind.

6. Verbindungen nach Anspruch 1 bis 4, dadurch gekennzeichnet, daß die reaktiven Derivate als Säurehalogenide, Säureanhydride, leicht zur Säure hydrolysierende Säureamide, Säurenitrile oder Säureester vorliegen.

7. Verbindungen nach Anspruch 1 bis 6, dadurch gekennzeichnet, daß die oligomeren oder prepolymeren Grundgerüste Homo-oder Copolymerisate von ethylenisch ungesättigten Monomeren sind.

8. Verbindungen nach Anspruch 1 bis 6, dadurch gekennzeichnet, daß die oligomeren oder polymeren Grundgerüste Polyester, Polyamide, Polyether, Polysulfone, Polyphosphazene oder Polysaccharide sind.

9. Verbindungen nach Anspruch 1 bis 8, dadurch gekennzeichnet, daß sie außer den Säure- und polymerisierbaren Gruppen Aldehyd-, Epoxid-, Isocyanat-oder Halotriazingruppen enthalten.

10. Verbindungen nach Anspruch 1 bis 9, dadurch gekennzeichnet, daß sie ein Molekulargewicht von mindestens 500 aufweisen.

11. Verbindungen nach Anspruch 1 bis 9, dadurch gekennzeichnet, daß sie ein Molekulargewicht von mindestens 1.500 aufweisen.

12. Verbindungen nach Anspruch 1 bis 11, dadurch gekennzeichnet, daß sie ein Molekulargewicht von maximal 100.000 aufweisen.

13. Verbindungen nach Anspruch 1 bis 11, dadurch gekennzeichnet, daß sie ein Molekulargewicht von maximal 20.000 aufweisen.

14. Poly(meth-)acrylierte Poly(meth-)acrylsäure.

15. Poly(meth-)acrylierte Oligomaleinsäure.

16. Poly(meth-)acrylierte Polymaleinsäure.

17. Poly(meth-)acrylierte Polycarbon-polyphosphonsäure.

18. Poly(meth-)acrylierte Polychlorophosphorsäure.

19. Poly(meth-)acryliertes Polysulfonat.

20. Poly(meth-)acrylierte Polyborsäure.

21. Poly(meth-)acrylierte Polyaldehydopolymaleinsäure.

22. Polymerisierbare Mischung, die eine oder mehrere Verbindungen nach den Ansprüchen 1 bis 21 enthält.

23. Polymerisierbare Mischung nach Anspruch 22, dadurch gekennzeichnet, daß sie zusätzlich andere polymerisierbare ungesättigte Verbindungen enthält.

24. Polymerisierbare Mischung nach Anspruch 22 bis 23, dadurch gekennzeichnet, daß sie zusätzlich Verbindungen mit Aldehyd-, Epoxid-, Isocyanat-oder Halotriazingruppen enthalten.

25. Polymerisierbare Mischung nach Anspruch 22 bis 24, dadurch gekennzeichnet, daß sie zusätzlich andere Verbindungen mit Säuregruppen deren Salzen oder deren reaktiven Derivaten enthält.

26. Polymerisierbare Mischung nach Anspruch 22 bis 25, dadurch gekennzeichnet, daß sie zusätzlich ein Lösungsmittel enthält.

27. Polymerisierbare Mischung nach Anspruch 22 bis 26, dadurch gekennzeichnet, daß sie zusätzlich Polymerisationskatalysatoren enthält.

28. Polymerisierbare Mischung nach Anspruch 22 bis 27, dadurch gekennzeichnet, daß sie gegebenenfalls oberflächenbehandelten, anorganischen oder organischen Füllstoff enthält.

29. Verwendung von polymerisierbaren Mischungen nach Anspruch 22 bis 28 als haftendes polymerisierbares Versiegelungs-oder Füllungsmaterial auf oxidischem, mineralischem, keramischem, glasartigem, metallischem oder biologischem Substrat.

30. Verwendung von polymerisierbaren Mischungen nach Anspruch 22 bis 28 als haftvermittelnde polymerisierbare Schicht zwischen oxidischem, mineralischem, keramischem, glasartigem, metallischem oder biologischem Substrat und einem polymerisierbaren Kunststoffmaterial, auf der Basis von Verbindungen mit ungesättigten Gruppen.

31. Verwendung von polymerisierbaren Mischungen nach Anspruch 22 bis 28 als Klebstoff für oxidische, mineralische, keramische, glasartige, metallische oder biologische Substrate.

32. Verwendung von polymerisierbaren Mischungen nach Anspruch 22 bis 28 zur Oberflächenbehandlung von Füllstoffen vor ihrer Einarbeitung in die Harzmatrix, zum besseren Verbund mit der sie umgebenden polymerisierbaren Harzmatrix.

33. Verwendung von polymerisierbaren Mischungen nach Anspruch 22 bis 28 zum Herstellen von Formkörpern.

34. Verwendung von polymerisierbaren Mischungen nach Anspruch 22 bis 28 zur Herstellung von polymerisierten auf Pigmenten und Füllstoffteilchen haftenden Überzügen.

35. Verwendung von polymerisierbaren Mischungen nach Anspruch 1 bis 34 für dentale und medizinische Zwecke.